# EUROPEAN PATENT APPLICATION

(11) **EP 4 759 258 A1**
(43) Date of publication of application: **17.06.2026**
(21) Application number: 25216407.4
(22) Date of filing: 17.11.2025
(51) Int. Cl.: A61B 18/14, A61B 18/00, A61M 25/00

(54) **SHAFT ASSEMBLY FOR CATHETER INSTRUMENT**

(30) Priority: 13.12.2024 US 202418980648
(71) Applicant: Biosense Webster (Israel) Ltd., 2066717 Yokneam (IL)
(72) Inventor: MOLINA-GONZALEZ, Edwin, Irvine, 92618 (US); MANRIQUEZ, Armida, Irvine, 92618 (US); PADILLA, Ricardo JR., Irvine, 92618 (US); BAH, Abubakarr, Irvine, 92618 (US); GARCIA ARMENDARIZ, Mario A., Irvine, 92618 (US); SIMMA, Anette A., Irvine, 92618 (US)
(74) Representative: Carpmaels & Ransford LLP

(57) **Abstract**

A catheter assembly includes a body and an elongate shaft extending distally from the body. The elongate shaft defines a lumen. The catheter assembly also includes an end effector extending distally from the elongate shaft, and at least one actuator element extending through the lumen. The at least one actuator element is configured to apply a proximally-directed force to at least one of the end effector or the elongate shaft. The catheter assembly further includes at least one flexible sleeve disposed about the at least one actuator element. The at least one flexible sleeve has a proximal end fixed against proximal movement relative to the body. The proximal end is proximal of the elongate shaft.

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively applying electrical energy to cardiac tissue, it may be possible to cease or modify the propagation of unwanted electrical signals from one portion of the heart to another. Some such ablation treatments may include radio frequency (RF) ablation with alternating current (AC) electrical energy; and/or irreversible electroporation (IRE) via pulsed field direct current (DC) electrical energy. The ablation process may provide a barrier to unwanted electrical pathways by creating electrically insulative lesions or scar tissue that effectively block communication of aberrant electrical signals across the ablated tissue.

In some procedures, a catheter with one or more electrodes may be used to provide ablation within a patient. The catheter may be inserted into a major vein or artery (e.g., the femoral artery) and then advanced to position the electrodes within the heart or in a structure adjacent to the heart (e.g., the pulmonary vein). The one or more electrodes may be placed in contact with cardiac tissue or other vascular tissue and then activated with electrical energy to thereby ablate the contacted tissue (e.g., via RF energy, IRE, etc.). In some cases, the electrodes may be bipolar. In some other cases, a monopolar electrode may be used in conjunction with a ground pad or other reference electrode that is in contact with the patient. Irrigation may be used to draw heat from components of an ablation catheter; and to prevent the formation of blood clots near the tissue treatment site.

Examples of ablation catheters are described in U.S. Pat. No. 10,743,932, entitled "Integrated Ablation System using Catheter with Multiple Irrigation Lumens," issued August 18, 2020, the disclosure of which is incorporated by reference herein, in its entirety; U.S. Pat. No. 10,660,700, entitled "Irrigated Balloon Catheter with Flexible Circuit Electrode Assembly," issued May 26, 2020, the disclosure of which is incorporated by reference herein, in its entirety; U.S. Pat. No. 11,559,349, entitled "Ablation Catheter with a Flexible Printed Circuit Board," issued January 24, 2023, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

Some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a top plan view of another example of a catheter assembly that may be used in the medical procedure of FIG. 1;
FIG. 3 depicts a side cross-sectional view of a junction of a catheter body and an intermediate section of the catheter assembly of FIG. 1, taken along a first diameter;
FIG. 4 depicts a side cross-sectional view of the junction of FIG. 3, taken along a second diameter generally perpendicular to the first diameter;
FIG. 5 depicts a perspective view of another example of a catheter assembly that may be used in the medical procedure of FIG. 1;
FIG. 6 depicts a partially exploded perspective view of the catheter assembly of FIG. 5, showing various internal components including a shaft-supporting sled and a coil anchor block;
FIG. 7 depicts a front elevation view of the coil anchor block of FIG. 6;
FIG. 8 depicts a front elevation view of the shaft-supporting sled of FIG. 6;
FIG. 9 depicts a top cross-sectional view of the catheter assembly of FIG. 5, taken along line 9-9 in FIG. 5;
FIG. 10A depicts a side cross-sectional view of the catheter assembly of FIG. 5, taken along line 10-10 in FIG. 5, with the shaft-supporting sled in a distal position; and
FIG. 10B depicts a side cross-sectional view of the catheter assembly of FIG. 5, taken along line 10-10 in FIG. 5, with the shaft-supporting sled in a proximal position.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

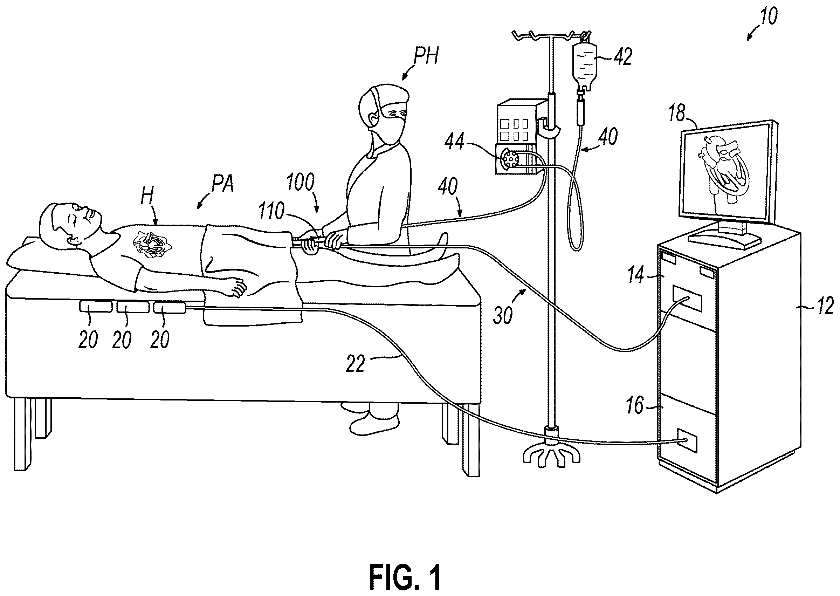

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector of a catheter (not shown) of catheter assembly (100) disposed in a patient (PA) to map potentials in tissue and/or ablate tissue in or near the heart (H) of the patient (PA).

Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via the electrodes of the end effector. Console (12) includes a processor (not shown) that processes such EP mapping signals and thereby provides EP mapping as is known in the art. In some other versions, the end effector includes other electrodes that are configured to provide EP mapping signals. In still other versions, the end effector does not provide EP mapping.

First driver module (14) of the present example is further operable to provide electrical power to the electrodes of the end effector, as will be described in greater detail below, to thereby ablate tissue. In some other versions, the end effector includes other electrodes that are configured to provide ablation. In still other versions, the end effector does not provide ablation.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (not shown) in the catheter near the end effector. In such versions, the processor of console (12) is also operable to process the position indicative signals from the navigation sensor assembly to thereby determine the position of the end effector within the patient (PA). In some versions, the navigation sensor assembly includes two or more coils that are operable to generate signals that are indicative of the position and orientation of the end effector within the patient (PA). The coils are configured to generate electrical signals in response to the presence of an alternating electromagnetic field generated by field generators (20). Other components and techniques that may be used to generate real-time position data associated with the end effector may include wireless triangulation, acoustic tracking, optical tracking, inertial tracking, and the like. While the navigation sensor assembly is shown as being disposed in the distal end of the catheter, the navigation sensor assembly may instead be positioned in the end effector. Alternatively, the catheter and the end effector may lack a navigation sensor assembly.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of the end effector. For instance, as the end effector of the catheter moves within the patient (PA), the corresponding position data from the navigation sensor assembly may cause the processor of console (12) to update the patient anatomy views in display (18) in real time to depict the regions of patient anatomy around the end effector as the end effector moves within the patient (PA). Moreover, the processor of console (12) may drive display (18) to show locations of aberrant conductive tissue sites, as detected via electrophysiological (EP) mapping with the end effector or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of the end effector on the images of the patient's anatomy, such as by superimposing an illuminated dot, a crosshair, a graphical representation of the end effector, or some other form of visual indication. Such a superimposed visual indication may also move within the images of the patient anatomy on display (18) in real time as the physician moves the end effector within the patient (PA), thereby providing real-time visual feedback to the operator about the position of the end effector within the patient (PA) as the end effector moves within the patient (PA). The images provided through display (18) may thus effectively provide a video tracking the position of the end effector within a patient (PA), without necessarily having any optical instrumentation (i.e., cameras) viewing the end effector. In the same view, display (18) may simultaneously visually indicate the locations of aberrant conductive tissue sites detected through EP mapping. The physician (PH) may thus view display (18) to observe the real time positioning of the end effector in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (not shown) of the end effector. Such irrigation may be provided in any suitable fashion as will be apparent to those skilled in the art in view of the teachings herein.

### II. Example of a Catheter with a Mapping Assembly

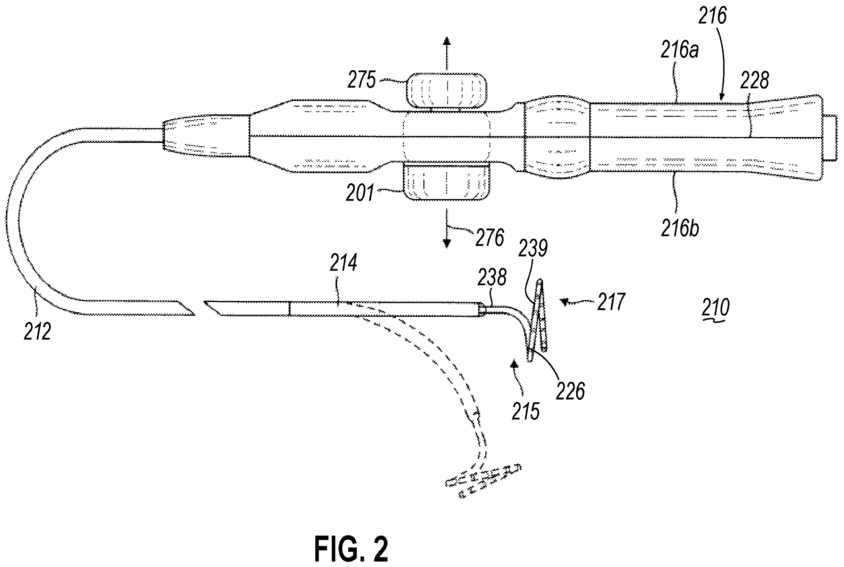

FIGS. 2-4 show an example of a catheter assembly (210) with multiple control capabilities for mapping and/or ablation of the heart that may be used in place of catheter assembly (100). In the example shown, catheter (210) comprises an elongate catheter body (212), a deflectable intermediate section (214) at a distal end of catheter body (212), a tip section (215) including an end effector in the form of a mapping assembly (217) at a distal end of intermediate section (214), and a multi-functional control handle (216) at a proximal end of catheter body (212) for controlling portions of catheter assembly (210) such as, for example, deflecting intermediate section (214) and contracting mapping assembly (217). Catheter body (212) and intermediate section (214) may collectively define an elongate catheter shaft.

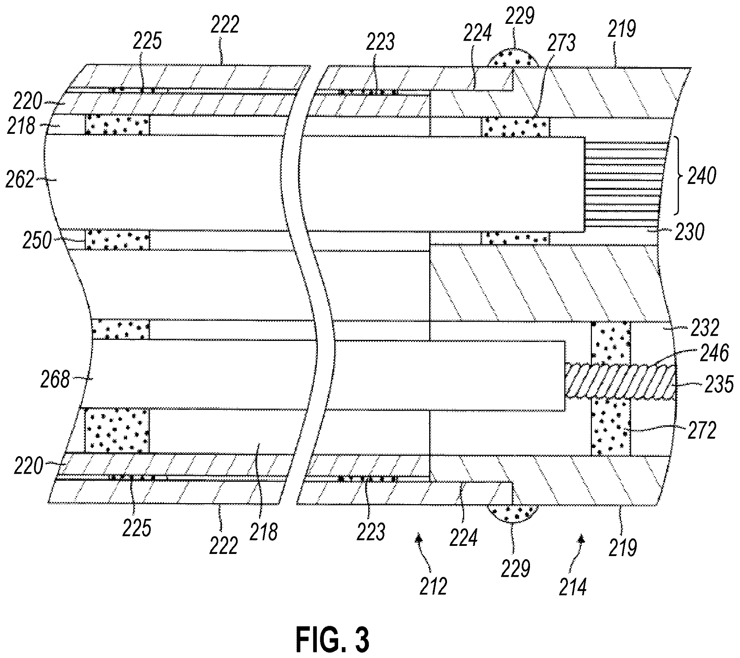

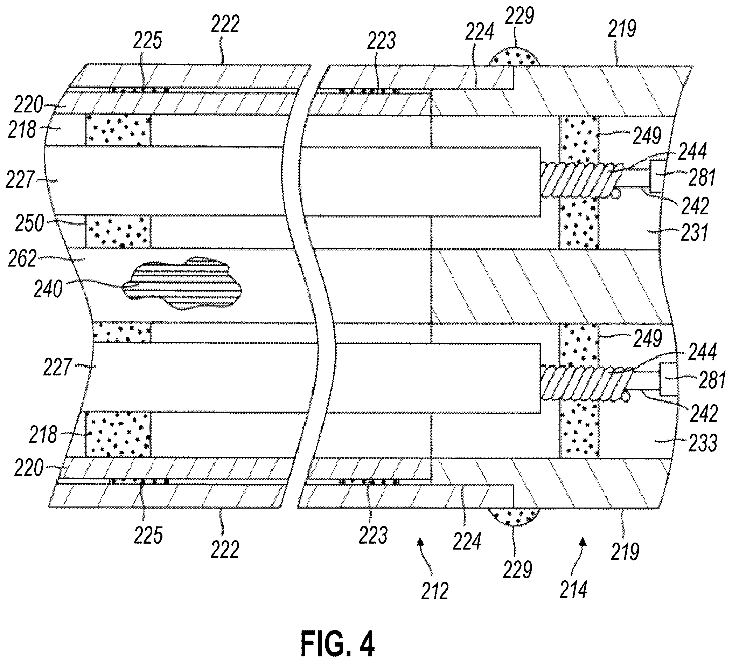

As shown in FIGS. 3 and 4, catheter body (212) comprises a single, central or axial lumen (218). In the example shown, catheter body (212) includes an outer wall (222) comprising an imbedded braided mesh of stainless steel or the like to increase torsional stiffness of catheter body (212) so that, when control handle (216) is rotated, tip section (215) may rotate in a corresponding manner. The inner surface of outer wall (222) is lined with a stiffening tube (220). A first glue joint (223) is made between the distal ends of the stiffening tube (220) and outer wall (222), and a second glue joint (225) is formed between the proximal ends of stiffening tube (220) and outer wall (222).

Intermediate section (214) comprises a shorter section of tubing (219) with multiple off-axis lumens, for example, first, second, third and fourth lumens (230, 231, 232, 233). The proximal end of intermediate section (214) comprises an inner counter bore (224) that receives the outer surface of polyimide stiffener (220). Intermediate section (214) and catheter body (212) are attached by a glue joint (229).

As shown in FIGS. 3 and 4, extending through lumen (218) of catheter body (212) are various components, for example, lead wires and multiple actuator elements in the form of puller members. Longitudinal movement of the puller members relative to catheter body (212) enable user control of various parts of catheter assembly (210) via control handle (216). In the example shown, the puller members include a pair of deflection puller members (242) for deflecting intermediate section (214) and a contraction puller member (235) for adjusting mapping assembly (217) of tip section (215).

Deflection puller member (242) extends through central lumen (218) of catheter body (212) and into second lumen (231) of intermediate section (214). Another deflection puller member (242) extends through central lumen (218) and into fourth lumen (233) of intermediate section (214). The distal ends of deflection puller members (242) may be anchored to the wall of tubing (219) near the distal end of intermediate section (214), such as by means of T-anchors (not shown). In intermediate section (214), each deflection puller member (242) extends through a plastic sheath (281), which may prevent deflection puller members (242) from cutting into the wall of tubing (219) of intermediate section (214) when intermediate section (214) is deflected.

As shown in FIG. 4, flexible sleeves in the form of compression coils (244) in surrounding relation to corresponding deflection puller members (242) extend from the proximal end of catheter body (212) to the proximal end of intermediate section (214). Compression coils (244) are made of any suitable metal, e.g., stainless steel. Each compression coils (244) is tightly wound on itself to provide flexibility, e.g., bending, but to resist compression. The inner diameters of compression coils (244) may be slightly larger than the diameters of the corresponding puller wires (242). For example, when a puller member (242) has a diameter of about 0.007 inches, the corresponding compression coil (244) may have an inner diameter of about 0.008 inches. A suitable coating on each puller member (242) may allow them to slide freely within the corresponding compression coils (244). The outer surface of each compression coil (244) is covered by a flexible, non-conductive sheath (227) to prevent contact between the compression coils (244) and other components, such as lead wires and cables, etc. In the example shown, compression coils (244) are anchored at their proximal ends to the proximal end of stiffening tube (220) in catheter body (212) by glue joint (250) and at their distal ends near the proximal end of intermediate section (214) in second lumen (231) and fourth lumen (233) by glue joints (249).

With reference to FIG. 2, at the distal end of intermediate section (214) is mapping assembly (217). Mapping assembly (217) comprises a generally straight proximal region (238) and a generally circular main region (239). Generally circular main region (239) can form a flat circle or can be at least slightly helical. Mapping assembly (217) is formed of a non-conductive cover or tubing (252) which can have any cross-sectional shape as desired. A series of ring electrodes (226) are mounted on non-conductive cover (252) of generally circular main region (239) of mapping assembly (217).

Contraction puller member (235), for example, a contraction puller wire, is provided to contract generally circular main region (239) to thereby change or reduce its diameter, for example, when mapping or ablating circular or tubular regions of the heart. Contraction wire (235) has a proximal end anchored in control handle (216), which is used to manipulate contraction wire (235) as described further below. Contraction wire (235) extends through central lumen (218) of catheter body (212), through third lumen (232) of intermediate section (214) and into non-conductive cover (252) of mapping assembly (217). The portion of contraction wire (235) extending through non-conductive cover (252) may be positioned on the side of generally circular main region (239) closer to the center of generally circular main region (239) to provide contraction of generally circular region (239).

As shown in FIG. 3, a flexible sleeve in the form of a third compression coil (246) is situated within catheter body (212) and intermediate section (214) in surrounding relation to contraction wire (235). Third compression coil (246) extends from the proximal end of catheter body (212) and to near the distal end of third lumen (232) of intermediate section (214). Third compression coil (246) is made of any suitable metal, such as stainless steel, and is tightly wound on itself to provide flexibility, e.g., bending, but to resist compression. The inner diameter of third compression coil (246) may be slightly larger than the diameter of contraction wire (235). The outer surface of compression coil (246) is covered by a flexible, non-conductive sheath (268), e.g., made of polyimide tubing. Third compression coil (246) is anchored at its proximal end to stiffening tube (220) of catheter body (212) by proximal glue joint (250) and to intermediate section (214) by distal glue joint (272).

Lead wires (240) attached to ring electrodes (226) extend through first lumen (230) of intermediate section (214), through central lumen (218) of catheter body (212), through control handle (216), and terminate at their proximal end in a connector (not shown) which is connected to an appropriate monitor or other device for receiving and displaying the information received from ring electrodes (226). The portion of lead wires (240) extending through central lumen (218) of catheter body (212), control handle (216), and the proximal end of intermediate section (214) is enclosed within a protective sheath (262). Protective sheath (262) is anchored at its distal end to the proximal end of intermediate section (214) by gluing it in lead wire lumen (230) with polyurethane glue or the like to form glue joint (273).

With reference to FIG. 2, control handle (216) comprises a generally elongate handle housing, including two opposing halves (216a, 216b) that generally mirror each other and are joined by glue, sonic welding or other suitable means along a longitudinal peripheral seam (228) around the housing. Control handle (216) houses components of a deflection control assembly including a deflection member or arm (275) that can be directly manipulated by an operator to control deflection of intermediate section (214). Deflection arm (275) is rotatable about an axis (276) that is generally transverse or perpendicular to the longitudinal axis of control handle (216). The deflection control assembly may have a rotatable rocker member (not shown) that acts on deflection puller members (242) to deflect intermediate section (214). Opposing deflection arm (275) is a deflection tension adjustment member or dial (201) which is coupled to and indirectly engaged with the rocker member by various mechanisms and parts and allows an operator to adjust the ease with which deflection arm (275) can be rotated.

For adjusting mapping assembly (217) by means of a third puller member, e.g., contraction wire (235), an end of contraction wire (235) extending between deflection puller members (242) within control handle (216) is anchored in control handle (216) for actuation by means of a linear control assembly (not shown) housed in control handle (216). By way of example only, catheter assembly (210), including such a linear control assembly of catheter assembly (210), may be constructed and operable in accordance with at least some of the teachings of U.S. Pat. No. 8,747,351, entitled "Catheter with multi-functional control handle having linear mechanism," issued June 10, 2014, the disclosure of which is incorporated by reference herein. In some versions, catheter assembly (210) is configured and operable like the VARIPULSE^{™} catheter by Biosense Webster, Inc. of Irvine, California.

### III. Example of Catheter Assembly with Translatable Shaft and Coil Anchor Block

In some instances, it may be desirable to provide a version of catheter assembly (210) that reduces or eliminates any potential risk of catheter body (212) buckling. For example, it may be desirable to provide a version of catheter assembly (210) that prevents or inhibits application of shearing-induced, longitudinally-compressive loads to catheter body (212) by any one or more of coils (244, 246). In addition, or alternatively, it may be desirable to provide a version of catheter assembly (210) in which the proximal ends of coils (244, 246) are mechanically grounded to control handle (216) with reduced reliance on glue, which may become brittle from exposure to heat (e.g., during sterilization of catheter assembly (210)) and thus may become vulnerable to cracking (e.g., during bending of catheter body (212)). For example, it may be desirable to omit glue joints (250) and to provide mechanical grounding of the proximal ends of coils (244, 246) to control handle (216) in a manner that bypasses catheter body (212). In some instances, it may be desirable to further protect catheter body (212) from other types of longitudinally-compressive loads that might otherwise be applied to catheter body (212) by any one or more of coils (244, 246). For example, it may be desirable to prevent or inhibit application of longitudinally-compressive loads to catheter body (212) by any one or more of coils (244, 246) that might otherwise result from an effective shortening of one or more coils (244, 246) (e.g., due to buckling of one or more coils (244, 246)).

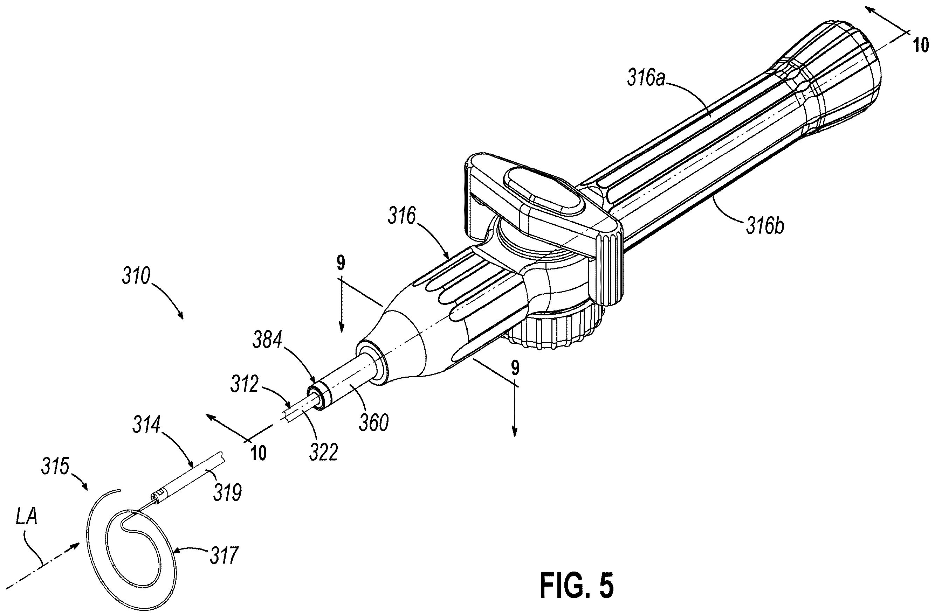

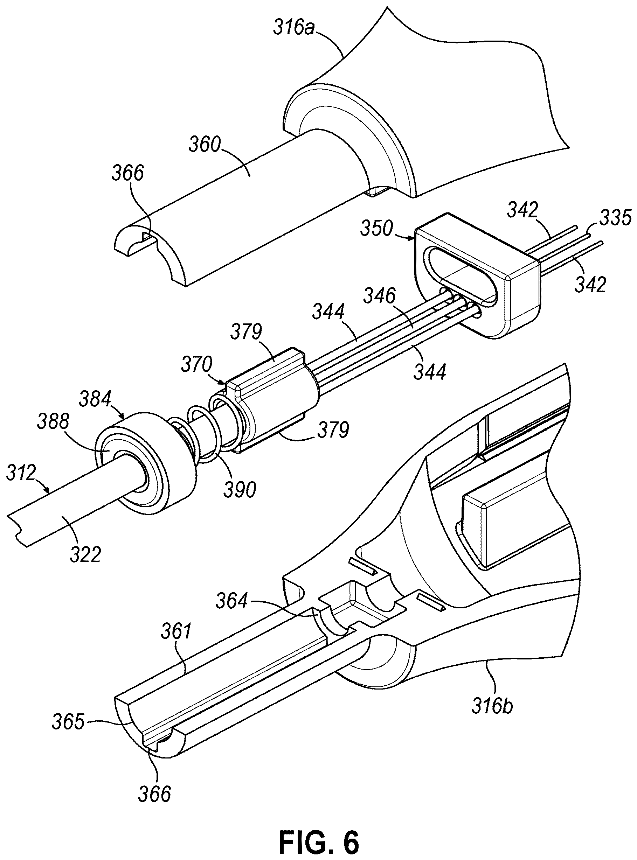

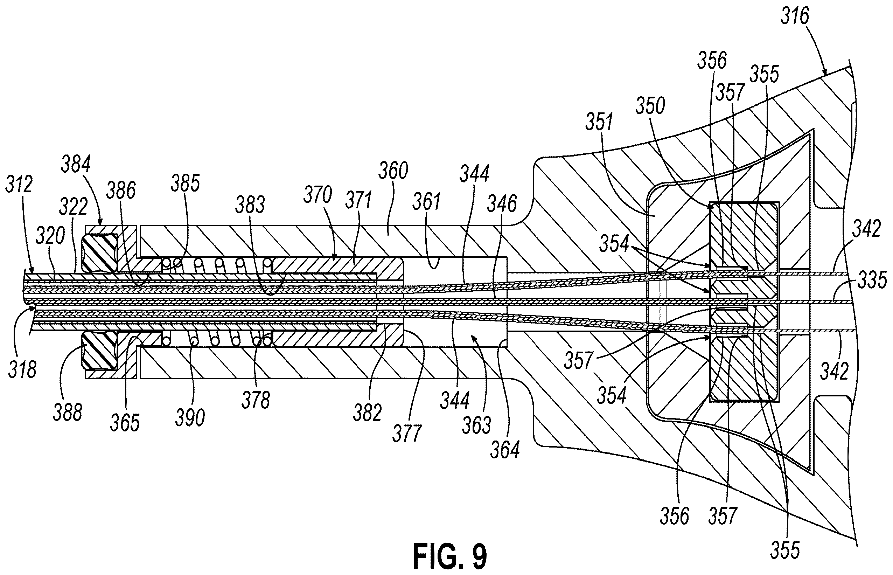

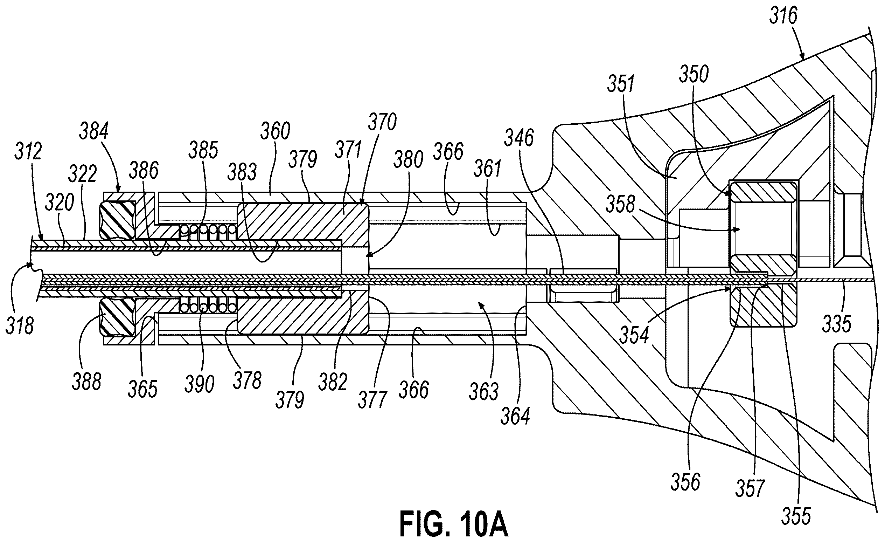

FIGS. 5-10B show an example of a catheter assembly (310) that may provide at least some, if not all, of the features and functionalities described above. Catheter assembly (310) may be similar to catheter assembly (210) described above, except as otherwise described below. In this regard, catheter assembly (310) of the present example includes an elongate catheter body (312), a deflectable intermediate section (314) at a distal end of catheter body (312), a tip section (315) including an end effector in the form of a mapping assembly (317) at a distal end of intermediate section (314), and a control handle (316) at a proximal end of catheter body (312). Catheter body (312) and intermediate section (314) may collectively define an elongate catheter shaft. Control handle (316) comprises a generally elongate handle housing, including two opposing halves (316a, 316b). As best shown in FIGS. 9 and 10A-10B, catheter body (312) comprises a central lumen (318), an outer wall (322), and a stiffening tube (320). As shown in FIG. 5, intermediate section (314) comprises tubing (319).

As best shown in FIG. 9, extending through lumen (318) of catheter body (312) are multiple actuator elements and, more particularly, a pair of deflection puller members in the form of puller wires (342) for deflecting intermediate section (314) away from a longitudinal axis (LA) defined by catheter body (312), and a contraction puller member in the form of a puller wire (335) for adjusting mapping assembly (317) of tip section (315). The distal ends of deflection puller members (342) may be anchored to the wall of tubing (319) near the distal end of intermediate section (314), such as by means of T-anchors (not shown), while contraction puller member (335) may extend all the way to mapping assembly (317). A pair of flexible sleeves in the form of compression coils (344) are positioned about corresponding deflection puller members (342), and a flexible sleeve in the form of a third compression coil (346) is positioned about contraction wire (335). The outer surface of each compression coil (344, 346) may be covered by a respective flexible, non-conductive sheath (not shown), such as a respective sheath (227, 268) described above.

In the example shown, compression coils (344, 346) are not anchored at their proximal ends to stiffening tube (320) or outer wall (322) of catheter body (312) by a glue joint. Thus, compression coils (344, 346) are not adhered to catheter body (312) at their proximal ends. In some versions, compression coils (344, 346) may not be adhered to catheter body (312) at any locations along their respective lengths, such that compression coils (344, 346) may be considered decoupled from catheter body (312) along their entire respective lengths. Rather, compression coils (344, 346) of the present example each extend proximally beyond catheter body (312) into control handle (316), where compression coils (344, 346) are each mechanically grounded to control handle (316).

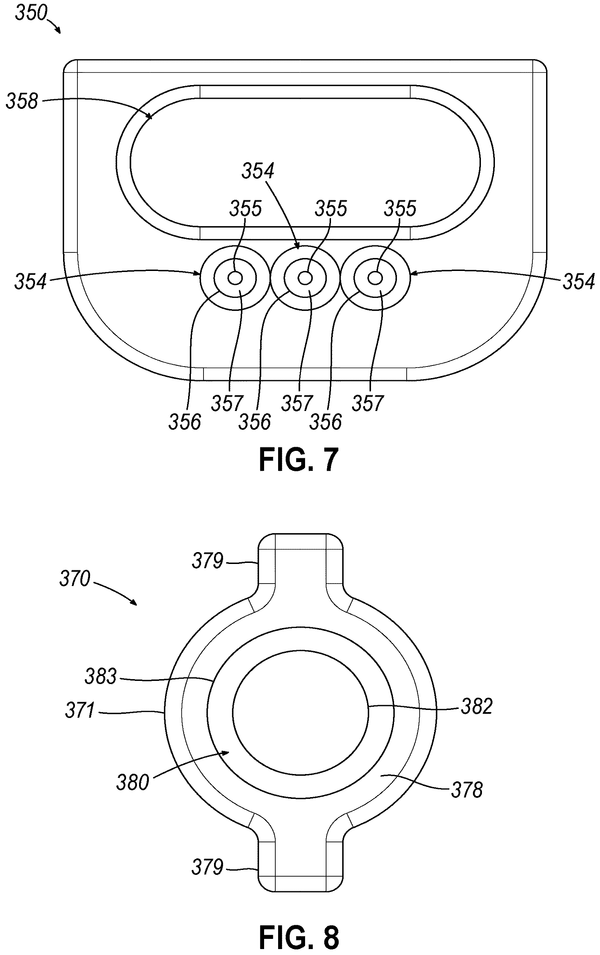

In this regard, control handle (316) of the present example includes an anchor block (350) fixedly secured to one or both housing halves (316a, 316b) within an interior of control handle (316). In the example shown, anchor block (350) is fixedly secured to upper housing half (316a) via a bracket (351). As best shown in FIG. 7, anchor block (350) includes a plurality of (e.g., three) longitudinally-extending bores (354), each including a respective generally cylindrical proximal bore portion (355) and a respective generally cylindrical distal bore portion (356). Each proximal bore portion (355) is narrower than the respective distal bore portion (356), such that each proximal bore portion (355) cooperates with the respective distal bore portion (356) to define a respective distally-facing, generally annular shoulder (357).

Each proximal bore portion (355) is sized and configured to slidably receive a corresponding puller wire (335, 342) to thereby permit actuation of the corresponding puller wire (335, 342), but has a cross-sectional dimension (e.g., diameter) that is less than that of the corresponding compression coil (344, 346) such that the corresponding compression coil (344, 346) may not be received therein. Rather, the corresponding compression coil (344, 346) may be received within the respective distal bore portion (356) and seated against the respective shoulder (357). In this manner, the respective shoulder (357) may define a proximal hard stop that is configured to restrict (e.g., prevent) longitudinal movement of the corresponding compression coil (344, 346) relative to anchor block (350), at least in the proximal direction, such that each compression coil (344, 346) is fixed against proximal movement relative to control handle (316). In the example shown, deflection puller wires (342) are received within respective laterally outer proximal bore portions (355), and compression coils (344) are received within respective laterally outer distal bore portions (356); while contraction puller wire (335) is received within the central proximal bore portion (355), and compression coil (346) is received within the central distal bore portion (356). In some cases, a proximal end of each compression coil (344, 346) may be adhered or otherwise affixed to the respective shoulder (357) to further fix each compression coil (344, 346) against distal movement relative to control handle (316). As shown, anchor block (350) also includes an oblong aperture (358), which may be sized and configured to permit routing of various other components, such as lead wires and cables, therethrough.

Thus, the proximal ends of compression coils (344, 346) are each mechanically grounded to control handle (316) via anchor block (350), rather than via catheter body (312). Such a configuration may prevent or inhibit application of shearing-induced, longitudinally-compressive loads to catheter body (312) by any one or more of coils (344, 346) and thereby reduce or eliminate any potential risk of catheter body (312) buckling.

In the example shown, catheter body (312) is dynamically coupled to control handle (316) to further reduce or eliminate any potential risk of catheter body (312) buckling. More particularly, catheter body (312) of the present example is configured to translate relative to control handle (316) through a predetermined range of longitudinal motion, such that proximally-directed forces applied to catheter body (312) that exceed a predetermined threshold magnitude may be converted into proximal translation of catheter body (312) rather than causing longitudinal compression of catheter body (312).

In this regard, control handle (316) further includes a distal neck portion (360) having a generally cylindrical bore (361) that at least partially defines a distal passageway (363). In the example shown, passageway (363) extends between a generally annular proximal end (364) and an open distal end (365), and further includes a pair of opposed (e.g., upper and lower) keyways (366) extending radially outwardly from bore (361). A proximal end of catheter body (312) is supported by a sled (370) that is configured to translate longitudinally within passageway (363), such that catheter body (312) is configured to translate longitudinally together with sled (370). Sled (370) of the present example includes a generally cylindrical body (371) extending between proximal and distal ends (377, 378) and configured to be slidably received within bore (361). Sled (370) further includes a pair of opposed (e.g., upper and lower) keys (379) extending radially outwardly from body (371) and configured to be slidably received within corresponding keyways (366), to thereby prevent rotation of sled (370) relative to neck portion (360) and thus maintain angular alignment of catheter body (312) with control handle (316). As shown, sled (370) also includes a longitudinally-extending central bore (380) having a generally cylindrical proximal bore portion (382) and a generally cylindrical distal bore portion (383) that is wider than proximal bore portion (382). Distal bore portion (383) is sized and configured to fixedly retain the proximal end of catheter body (312), such as by providing an interference fit therewith. Proximal bore portion (382) is sized and configured to permit routing of puller wires (335, 342) together with the respective compression coils (344, 346), and/or various other components, such as lead wires and cables, therethrough.

In the example shown, a handle cap (384) is secured to open distal end (365) to capture sled (370) within passageway (363), such that a proximal end (385) of handle cap (384) and proximal end (364) of passageway (363) may cooperate with each other to define a range of longitudinal motion of sled (370) relative to neck portion (360), and thus the range of longitudinal motion of catheter body (312) relative to control handle (316). Handle cap (384) of the present example includes a central bore (386) sized and configured to slidably receive catheter body (312) to thereby permit translation of catheter body (312). A gasket in the form of an O-ring (388) is coupled to handle cap (384) for providing a fluid-tight seal between handle cap (384) and catheter body (312) to prevent ingress of fluid (e.g., blood) into passageway (363).

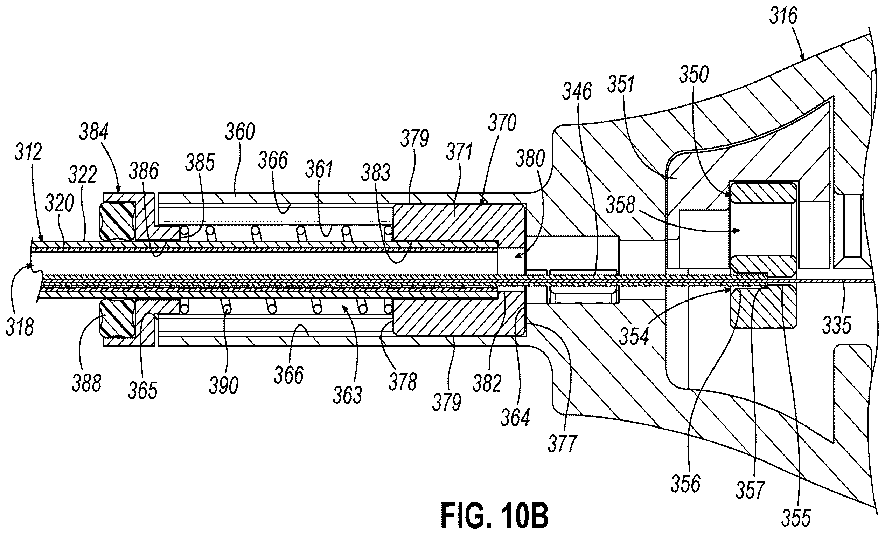

As shown, a resilient biasing member in the form of a helical tension spring (390) is positioned about catheter body (312) within bore (361) of passageway (363) and, more particularly, between distal end (378) of sled (370) and proximal end (385) of handle cap (384). Spring (390) is configured to resiliently bias sled (370) toward a distal position (FIG. 10A), and to impose a minimum threshold magnitude of proximally-directed force to be applied to sled (370) (e.g., via catheter body (312)) in order to translate sled (370) from the distal position toward a proximal position (FIG. 10B). The minimum threshold magnitude may be selected based on the various proximally-directed forces that may be expected to be applied to catheter body (312) during normal operation, and/or based on a column strength of catheter body (312). For example, the minimum threshold magnitude may be substantially greater than the magnitudes of any proximally-directed forces that may be applied to catheter body (312) by one or more puller wires (335, 342) during normal operation of puller wires (335, 342), such that sled (370) may be configured to remain in the distal position during normal operation of puller wires (335, 342). In addition, or alternatively, the minimum threshold magnitude may be substantially less than the magnitude of a proximally-directed force that would otherwise be sufficient to cause catheter body (312) to buckle based on the column strength of catheter body (312), such that sled (370) may be configured to translate toward the proximal position before a proximally-directed force applied to catheter body (312) can reach the magnitude that would be sufficient to cause catheter body (312) to buckle.

While tension spring (390) is shown in the present example, it will be appreciated that any other suitable resilient biasing member may be used in place of tension spring (390). For example, other versions may instead include a compression spring (not shown) positioned within passageway (363) between proximal end (377) of sled (370) and proximal end (364) of passageway (363) and configured to resiliently bias sled (370) toward the distal position.

In an example of a method of use, sled (370) may initially be in the distal position, such that catheter body (312) may be in an extended state relative to control handle (316), as shown in FIG. 10A. While sled (370) is in the distal position, various proximally-directed forces having magnitudes less than the minimum threshold magnitude may be applied to catheter body (312), and may thus be insufficient to overcome the resilient biasing of sled (370) toward the distal position. For example, such proximally-directed forces may be applied to catheter body (312) by one or more anatomical structures in the patient (PA) as catheter body (312) is moved through the patient (PA). As another example, such proximally-directed forces may be applied to catheter body (312) by one or more puller wires (335, 342). In this regard, the magnitude of any proximally-directed forces that might be applied to catheter body (312) by contraction wire (335) during contraction of mapping assembly (317) may be insufficient to overcome the resilient biasing of sled (370) toward the distal position, such that sled (370) may remain in the distal position and catheter body (312) may remain in the extended state. Similarly, the magnitude of any proximally-directed forces that might be applied to catheter body (312) by deflection puller wires (342) during deflection of intermediate section (314) may be insufficient to overcome the resilient biasing of sled (370) toward the distal position, such that sled (370) may remain in the distal position and catheter body (312) may remain in the extended state.

In some instances, one or more proximally-directed forces having a magnitude substantially equal to or greater than the minimum threshold magnitude may be applied to catheter body (312), and may thus be sufficient to overcome the resilient biasing of sled (370) toward the distal position. For example, such a proximally-directed force may be applied to catheter body (312) by one or more compression coils (344, 346) as a result of an effective shortening of one or more coils (344, 346) (e.g., due to buckling of one or more coils (344, 346)). Rather than potentially causing catheter body (312) to buckle, the application of such a proximally-directed force to catheter body (312) may cause sled (370) to translate proximally from the distal position toward the proximal position, such that catheter body (312) may retract from the extended state toward a retracted state relative to control handle (316), as shown in FIG. 10B. In response to the magnitude of such a proximally-directed force decreasing below the minimum threshold magnitude, the biasing of sled (370) by spring (390) may cause sled (370) to translate distally from the proximal position toward the distal position, such that catheter body (312) may extend from the retracted state toward the extended state relative to control handle (316), as shown in FIG. 10A.

### IV. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A catheter assembly, comprising: (a) a body; (b) an elongate shaft extending distally from the body, the elongate shaft defining a lumen; (c) an end effector extending distally from the elongate shaft; (d) at least one actuator element extending through the lumen, the at least one actuator element being configured to apply a proximally-directed force to at least one of the end effector or the elongate shaft; and (e) at least one flexible sleeve disposed about the at least one actuator element, the at least one flexible sleeve having a proximal end fixed against proximal movement relative to the body, the proximal end being proximal of the elongate shaft.

### Example 2

The catheter assembly of Example 1, further comprising an anchor block positioned within the body, the proximal end of the at least one flexible sleeve being fixed against proximal movement relative to the body via the anchor block.

### Example 3

The catheter assembly of Example 2, the anchor block including at least one distally-facing surface, the at least one flexible sleeve being seated against the at least one distally-facing surface.

### Example 4

The catheter assembly of Example 3, the anchor block including at least one bore having proximal and distal portions, the proximal portion being narrower than the distal portion, the at least one distally-facing surface including a generally annular shoulder defined by the proximal and distal portions of the bore.

### Example 5

The catheter assembly of Example 4, the at least one actuator element extending through the proximal and distal portions of the at least one bore.

### Example 6

The catheter assembly of any of Examples 1 through 5, the elongate shaft including an elongate catheter body and a deflectable intermediate section extending distally from the elongate catheter body.

### Example 7

The catheter assembly of Example 6, the at least one flexible sleeve being decoupled from the elongate catheter body along a length of the at least one flexible sleeve.

### Example 8

The catheter assembly of any of Examples 1 through 7, the elongate shaft being longitudinally translatable relative to the body between a distally extended position and a proximally retracted position.

### Example 9

The catheter assembly of Example 8, the elongate shaft being resiliently biased toward the distally extended position.

### Example 10

The catheter assembly of Example 9, the elongate shaft being resiliently biased toward the distally extended position by a spring.

### Example 11

The catheter assembly of any of Examples 8 through 9, the elongate shaft being fixed against rotation relative to the body.

### Example 12

The catheter assembly of any of Examples 1 through 11, the at least one actuator element including at least one puller wire.

### Example 13

The catheter assembly of any of Examples 1 through 12, the at least one flexible sleeve including at least one coil.

### Example 14

The catheter assembly of any of Examples 1 through 13, the at least one actuator element being configured to apply the proximally-directed force to the end effector to contract the end effector.

### Example 15

The catheter assembly of any of Examples 1 through 14, the at least one actuator element being configured to apply the proximally-directed force to the elongate shaft to deflect at least a distal portion of the elongate shaft.

### Example 16

The catheter assembly of any of Examples 1 through 15, the body including a control handle.

### Example 17

A catheter assembly, comprising: (a) a body; (b) an elongate shaft extending distally from the body, the elongate shaft defining a lumen; (c) an end effector extending distally from the elongate shaft; (d) at least one actuator element extending through the lumen, the at least one actuator element being configured to apply a proximally-directed force to at least one of the end effector or the elongate shaft; and (e) at least one flexible sleeve disposed about the at least one actuator element, the elongate shaft being longitudinally translatable relative to the body between a distally extended position and a proximally retracted position.

### Example 18

The catheter assembly of Example 17, further comprising a sled configured to translate longitudinally within a passageway of the body, the elongate shaft being fixedly secured to the sled.

### Example 19

The catheter assembly of Example 18, the sled being resiliently biased in a longitudinal direction.

### Example 20

The catheter assembly of Example 19, the sled being resiliently biased in the longitudinal direction by a spring.

### Example 21

The catheter assembly of any of Examples 17 through 20, the body including a control handle.

### Example 22

A catheter assembly, comprising: (a) a body; (b) an elongate shaft extending distally from the body, the elongate shaft defining a lumen; (c) an end effector extending distally from the elongate shaft; (d) at least one actuator element extending through the lumen, the at least one actuator element being configured to apply a proximally-directed force to at least one of the end effector or the elongate shaft; (e) at least one flexible sleeve disposed about the at least one actuator element, the at least one flexible sleeve having a proximal end; (f) an anchor block positioned within the body, the proximal end of the at least one flexible sleeve being fixed against proximal movement relative to the body via the anchor block; and (g) a sled configured to translate longitudinally within a passageway of the body, the elongate shaft being fixedly secured to the sled.

### Example 23

The catheter assembly of Example 22, the body including a control handle.

### V. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A catheter assembly, comprising:
(a) a body;
(b) an elongate shaft extending distally from the body, the elongate shaft defining a lumen;
(c) an end effector extending distally from the elongate shaft;
(d) at least one actuator element extending through the lumen, the at least one actuator element being configured to apply a proximally-directed force to at least one of the end effector or the elongate shaft; and
(e) at least one flexible sleeve disposed about the at least one actuator element, the at least one flexible sleeve having a proximal end fixed against proximal movement relative to the body, the proximal end being proximal of the elongate shaft.

2. The catheter assembly of claim 1, further comprising an anchor block positioned within the body, the proximal end of the at least one flexible sleeve being fixed against proximal movement relative to the body via the anchor block.

3. The catheter assembly of claim 2, the anchor block including at least one distally-facing surface, the at least one flexible sleeve being seated against the at least one distally-facing surface.

4. The catheter assembly of claim 3, the anchor block including at least one bore having proximal and distal portions, the proximal portion being narrower than the distal portion, the at least one distally-facing surface including a generally annular shoulder defined by the proximal and distal portions of the bore.

5. The catheter assembly of claim 4, the at least one actuator element extending through the proximal and distal portions of the at least one bore.

6. The catheter assembly of any preceding claim, the elongate shaft including an elongate catheter body and a deflectable intermediate section extending distally from the elongate catheter body, optionally the at least one flexible sleeve being decoupled from the elongate catheter body along a length of the at least one flexible sleeve.

7. The catheter assembly of any preceding claim, the elongate shaft being longitudinally translatable relative to the body between a distally extended position and a proximally retracted position.

8. The catheter assembly of claim 7, the elongate shaft being resiliently biased toward the distally extended position.

9. The catheter assembly of claim 8, the elongate shaft being resiliently biased toward the distally extended position by a spring.

10. The catheter assembly of claim 7 or claim 8, the elongate shaft being fixed against rotation relative to the body.

11. The catheter assembly of any preceding claim, the at least one actuator element including at least one puller wire.

12. The catheter assembly of any preceding claim, the at least one flexible sleeve including at least one coil and/or the at least one actuator element being configured to apply the proximally-directed force to the end effector to contract the end effector and/or to apply the proximally-directed force to the elongate shaft to deflect at least a distal portion of the elongate shaft.

13. A catheter assembly, comprising:
(a) a body;
(b) an elongate shaft extending distally from the body, the elongate shaft defining a lumen;
(c) an end effector extending distally from the elongate shaft;
(d) at least one actuator element extending through the lumen, the at least one actuator element being configured to apply a proximally-directed force to at least one of the end effector or the elongate shaft; and
(e) at least one flexible sleeve disposed about the at least one actuator element,
the elongate shaft being longitudinally translatable relative to the body between a distally extended position and a proximally retracted position.

14. The catheter assembly of claim 13, further comprising a sled configured to translate longitudinally within a passageway of the body, the elongate shaft being fixedly secured to the sled, optionally the sled being resiliently biased in a longitudinal direction, further optionally the sled being resiliently biased in the longitudinal direction by a spring.

15. A catheter assembly, comprising:
(a) a body;
(b) an elongate shaft extending distally from the body, the elongate shaft defining a lumen;
(c) an end effector extending distally from the elongate shaft;
(d) at least one actuator element extending through the lumen, the at least one actuator element being configured to apply a proximally-directed force to at least one of the end effector or the elongate shaft;
(e) at least one flexible sleeve disposed about the at least one actuator element, the at least one flexible sleeve having a proximal end;
(f) an anchor block positioned within the body, the proximal end of the at least one flexible sleeve being fixed against proximal movement relative to the body via the anchor block; and
(g) a sled configured to translate longitudinally within a passageway of the body, the elongate shaft being fixedly secured to the sled.
